# EUROPEAN PATENT APPLICATION

(11) **EP 2 067 856 A1**
(43) Date of publication of application: **10.06.2009**
(21) Application number: 07828384.3
(22) Date of filing: 26.09.2007
(51) Int. Cl.: C12N 7/00, C12M 3/00, C12Q 1/02, C12Q 1/70, G01N 33/15

(54) **METHOD OF PROLIFERATING HEPATITIS VIRUS, HOLLOW FIBER FOR CULTURING HEPATITIS VIRUS-INFECTED CELLS AND UTILIZATION OF THE SAME**

(30) Priority: 26.09.2006 JP 2006260088
(71) Applicant: Toyo Boseki Kabushiki Kaisha, Osaka-shi, Osaka 530-8230 (JP); Kyoto University, Kyoto-shi Kyoto 606-8501 (JP)
(72) Inventor: YAMAGUCHI, Tatsuya, Tsuruga-shi Fukui 914-0047 (JP); SEGAWA, Masaya, Tsuruga-shi Fukui 914-0047 (JP); MIZOKAMI, Masashi, Nagoya Aichi 467-8601 (JP); TANAKA, Yasuhito, Nagoya Aichi 467-8601 (JP); SHIMOTOHNO, Kunitada, Kyoto-shi Kyoto 606-8507 (JP); HIJIKATA, Makoto, Kyoto-shi Kyoto 606-8507 (JP)
(74) Representative: Vossius & Partner
(86) International application number: PCT/JP2007/068611
(87) International publication number: WO 2008/038641

(57) **Abstract**

The present invention provides (i) a method for proliferating a hepatitis virus, the method including the steps of: infecting, with the hepatitis virus, cells packed into a lumen of a hollow fiber made of a permeabilized membrane; and culturing the cells or (ii) a method for proliferating a hepatitis virus, the method including the steps of: infecting cells with the hepatitis virus; packing the cells into a lumen of a hollow fiber made of a permeabilized membrane; and culturing the cells. This provides an experimental system for infecting *in vitro* culture cells with a hepatitis virus such as HBV or HCV and then proliferating the hepatitis virus.

## Description

### Technical Field

The present invention relates to a method for proliferating a hepatitis virus such as hepatitis B virus (HBV) or hepatitis C virus (HCV), a hollow fiber for culturing hepatitis virus-infected cells, and use of a drug evaluation method that involves the use of the hollow fiber or the like.

### Background Art

Hepatitis B virus (HBV) is one of the most major causes of acute or chronic liver disease such as fulminant hepatitis, cirrhosis, or liver cancer in the world or, in particular, in Asian and African countries. According to the WHO (World Health Organization), it is estimated that 350 million people have been infected with HBV worldwide, and the number of people persistently infected with HBV in Japan is estimated to reach 1.5 million, including the number of asymptomatic carriers.

HBV is now treated with use of a therapeutic drug (e.g., lamibudine), interferon, or the like. However, the use of such a drug poses a serious problem with a high chance of emergence of drug-resistant strains of the virus. For this reason, there are many cases where no adequate therapeutic effect can be obtained, and there is a desire for the development of an effective antiviral drug.

HBV is known to transiently proliferate by forcibly transfecting its gene into an established cell line such as Huh7 cells or HepG2 cells (Non Patent Literature 1). However, there has existed no effective system for proliferating HBV by infecting human hepatocytes with the virus by contact. For this reason, basic researches and experiments in which the effect of a drug is studied have been conducted with use of chimpanzees or chimera mice into which human hepatocytes have been implanted. However, the use of such animals is limited because of high costs, problems with individual differences and reproducibility, and animal protection. In view of such a background, there has been a true desire for an effective experimental infection system, capable of stably proliferating HBV *in vitro,* which serves to realize early development of an effective therapeutic drug.

It is said that approximately 300 million people have been infected with hepatitis C virus (HCV) worldwide, and the number of people persistently infected with HCV in Japan is estimated to reach 2 millions. HCV carriers are highly likely to develop chronic hepatitis some of which are serious infectious diseases that develop into cirrhosis or liver cancer. According to the WHO, it is reported that 3 to 4 million people are newly infected with HCV every year. HCV is now treated with use of a combination of interferon and rivabirin, which is an antiviral drug. However, there has been an urgent need to develop a new vaccine for prevention of the spread of HCV infection and for virus eradication.

HCV is mainly treated with a combination of interferon and rivabirin. However, in Japan, the combined drug suffers from a low cure rate because there are many people infected with a type of hepatitis virus, such as HCV-1a or HCV-1b, against which interferon is less effective. Besides, interferon poses a problem with a strong side effect. In view of such a background, there is also a desire for the development of an antiviral drug effective against HCV.

HCV is known to infect only such living organisms as human beings and chimpanzees. In 1999, an HCV subgenome replicon system for replicating and proliferating the HCV subgenome in cultured cells was introduced. However, the technique is only capable of viral RNA replication. Besides, the number of strains whose viral RNA can be replicated is limited to a very small number. Replication of HCV particles with use of cultured cells was made possible for the first time in the world with use of a JFH-1 strain, which had been cloned by Wakita, et al. in 2005 (Non Patent Literature 2). However, there is a fatal limitation in that the JFH-1 strain was isolated from a patient with fulminant hepatitis, which is extremely rare for HCV infectious disease.

Ever since then, HBV or HCV infection and proliferation have been confirmed in normal human hepatocytes or cultured hepatocytes such as strains of human hepatocyte. However, such HBV or HCV infection is only transient, or exhibits only an extremely low infectivity titer. This makes it hard to say that any of the culture systems is practical, for example, for evaluation of the effectiveness of a drug.

One of the main reasons why a high viral infectivity cannot be obtained even when cultured hepatocytes have been infected is attributed to the way in which the cells are cultured. According to a monolayer culture method that involves the use of a culture dish or the like, cells are placed in an environment extremely different from an *in vivo* environment. This causes the cells to lose many of their functions. As a result, even when the cells are infected with a virus, the virus infection continues only for a short period of time. This makes it impossible to allow the virus to cause infection or proliferate with long-term stability.

For example, if a virus strain isolated from a patient with chronic HBV or HCV can be cultured in a simple manner so as to have a high infectivity titer, an effective antiviral drug can be screened *in vitro*. However, there has existed no such virus culture system.

### Citation List

Non Patent Literature 1
Sureau et al.: Cell, 47(1): pp. 37-47 (1986)
Non Patent Literature 2
Wakita, T. et al.: Virus, 55(2): pp. 287-296 (2005)

### Summary of Invention

The present invention is made in the view of the problem, and a main object of the present invention is to provide an experimental system for proliferating a hepatitis virus such as HBV or HCV by infecting *in vitro* cultured cells with the virus.

The present invention further provides a culture system that allows infection and proliferation of a hepatitis virus derived from a serum of an HBV- or HCV-infected patient, thereby making it possible to (i) carry out simple screening for the development of an effective antiviral drug and to (ii) predict the effect of a therapeutic drug on a hepatitis virus-infected patient.

In order to attain the object, the inventors of the present invention carried out a diligent study. As a result, they found that by culturing cells infected with a hepatitis virus and packed into a lumen of a hollow fiber made of a permeabilized membrane, the hepatitis virus can be stably proliferated *in vitro* with high efficiency. That is, the present invention provides a hepatitis virus proliferation method that makes it possible to stably proliferate a hepatitis virus *in vitro* with high efficiency by culturing hepatitis virus-infected cells in a lumen of a hollow fiber made of a permeabilized membrane.

Conventionally, human hepatocytes have been cultured by the monolayer culture method, which involves the use of a culture vessel such as a dish or a plate. However, as described above, the monolayer culture method can only yield transient infection or a low infectivity titer even when the hepatocytes have been infected with the virus. The inventors of the present invention found it possible to obtain a remarkably high virus infectivity titer by (i) packing, into the lumen of the hollow fiber, hepatitis virus-infected cells from which only a low infectivity titer could be obtained by the monolayer culture method and (ii) culturing the cells three-dimensionally.

The present invention further provides a screening method for evaluating the effect of a drug on a hepatitis virus with use of hepatitis virus-infected cells cultured as described above.

Further, the present invention may make it possible to identify an unknown HBV or HCV receptor of a HBV- or HCV-infected host cell. The present invention further provides a model important in studying natural history from infection to replication. As such, the present invention may be applied to the development of a drug targeted at a new host factor.

Specifically, the present invention includes the following arrangements.

### (Aspect 1)

A method for proliferating a hepatitis virus, the method including the steps of: infecting, with the hepatitis virus, cells packed into a lumen of a hollow fiber made of a permeabilized membrane; and culturing the cells.

### (Aspect 2)

A method for proliferating a hepatitis virus, the method including the steps of: infecting cells with the hepatitis virus; packing the cells into a lumen of a hollow fiber made of a permeabilized membrane; and culturing the cells.

### (Aspect 3)

The method as set forth in Aspect 1 or 2, further including the step of applying pressure or centrifugal force to the lumen of the hollow fiber into which the cells have been packed.

### (Aspect 4)

The method as set forth in any one of Aspects 1 through 3, wherein the cells are hepatocytes or liver-derived cells.

### (Aspect 5)

The method as set forth in Aspect 4, wherein the cells are HuS-E/2 cells (FERM ABP-10908).

### (Aspect 6)

The method as set forth in any one of Aspects 1 through 5, wherein the hepatitis virus is hepatitis B virus or hepatitis C virus.

### (Aspect 7)

A method for manufacturing a hollow fiber for culturing hepatitis virus-infected cells, the method including any of the following steps of: (i) infecting, with a hepatitis virus, cells packed into a lumen of a hollow fiber made of a permeabilized membrane; and (ii) infecting cells with a hepatitis virus and then packing the cells into a lumen of a hollow fiber made of a permeabilized membrane.

### (Aspect 8)

A hollow fiber for culturing hepatitis virus-infected cells, the hollow fiber being manufactured by a method as set forth in Aspect 7.

### (Aspect 9)

A method for producing a hepatitis vaccine, the method using a culture of hepatitis virus-infected cells, a hepatitis virus particle, or a constituent protein of a hepatitis virus particle, each being obtained by a method as set forth in any one of Aspects 1 through 6.

### (Aspect 10)

A kit for proliferating a hepatitis virus, the kit including a hollow fiber, made of a permeabilized membrane, whose lumen has been filled with hepatitis virus-infectable cells.

### (Aspect 11)

A method for screening a drug that inhibits hepatitis virus proliferation or hepatitis virus protein expression, the method including the step of culturing, together with a drug candidate substance that is to be evaluated, hepatitis virus-infected cells packed into a lumen of a hollow fiber made of a permeabilized membrane.

### (Aspect 12)

A method for screening a drug that inhibits hepatitis virus infection, the method including the step of culturing, together with a hepatitis virus and a drug candidate substance that is to be evaluated, hepatitis virus-infectable cells packed into a lumen of a hollow fiber made of a permeabilized membrane.

### (Aspect 13)

A kit for screening a drug that inhibits hepatitis virus infection, hepatitis virus proliferation, or hepatitis virus protein expression, the kit including a hollow fiber, made of a permeabilized membrane, whose lumen has been filled with hepatitis virus-infectable cells.

### (Aspect 14)

A method for screening a drug for treating a hepatitis virus-infected person, the method including the step of culturing, together with a drug candidate substance that is to be evaluated, cells (i) packed into a lumen of a hollow fiber made of a permeabilized membrane and (ii) infected with a hepatitis virus derived from a serum of the hepatitis virus-infected person.

### (Aspect 15)

A method for screening a drug for treating a hepatitis virus-infected person, the method including the step of culturing, together with (i) a hepatitis virus derived from a serum of the hepatitis virus-infected person and (ii) a drug candidate substance that is to be evaluated, hepatitis virus-infectable cells packed into a lumen of a hollow fiber made of a permeabilized membrane.

### (Aspect 16)

A method for finding a combination of drugs most suitable for treating a hepatitis virus-infected person, the method including the step of culturing, together with a drug candidate substance that is to be evaluated, hepatitis virus-infected cells packed into a lumen of a hollow fiber made of a permeabilized membrane.

### (Aspect 17)

A kit for finding a combination of drugs most suitable for treating a hepatitis virus-infected person, the kit including a hollow fiber, made of a permeabilized membrane, whose lumen has been filled with hepatitis virus-infectable cells.

### (Aspect 18)

The method as set forth in Aspect 16, wherein the hepatitis virus-infected cells are hepatitis virus-infected cells derived from a serum of the hepatitis virus-infected person.

### (Aspect 19)

A diagnostic or examination method for treating a hepatitis virus-infected person, the method including the step of culturing, together with a drug that is to be evaluated, hepatitis virus-infected cells packed into a lumen of a hollow fiber made of a permeabilized membrane.

For a fuller understanding of the nature and advantages of the invention, reference should be made to the ensuing detailed description taken in conjunction with the accompanying drawings.

### Brief Description of Drawings

Fig. 1

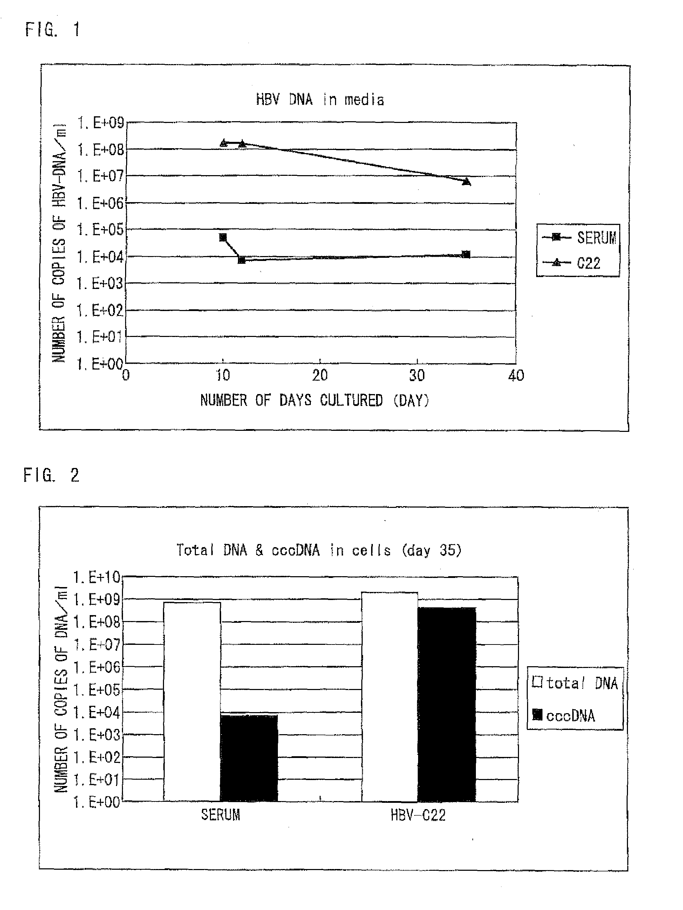

   Fig. 1 is a graph, obtained in Example 1 with use of the present invention, which shows transitions in the number of copies of HBV DNA in a culture supernatant obtained from a culture of cells that have been infected with a patent's serum-derived HBV and cultured to proliferate the HBV.
Fig. 2
   Fig. 2 is a graph, obtained in Example 1 with use of the present invention, which shows transitions in the number of copies of HBV cccDNA in cells infected with HBV and cultured to proliferate the HBV.
Fig. 3

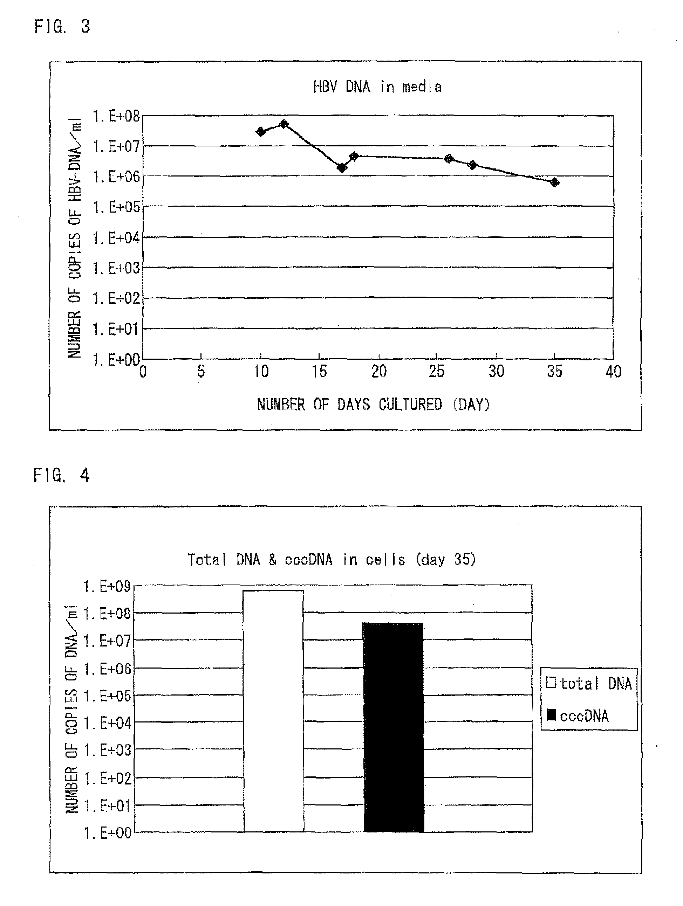

   Fig. 3 is a graph, obtained in Example 2 with use of the present invention, which shows transitions in the number of copies of HBV DNA in a culture supernatant obtained from a culture of cells that have been infected with HBV and cultured to proliferate the HBV.
Fig. 4
   Fig. 4 is a graph, obtained in Example 2 with use of the present invention, which shows transitions in the number of copies of HBV cccDNA in cells infected with HBV and cultured to proliferate the HBV.
Fig. 5

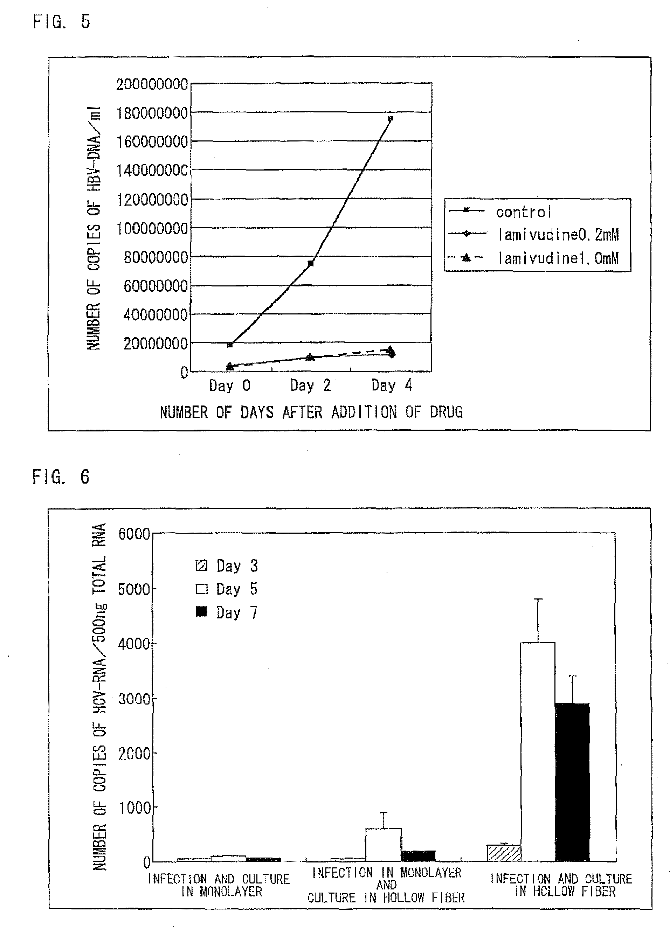

   Fig. 5 is a graph, obtained in Example 3 with use of the present invention, which shows transitions in the number of copies of HBV DNA in a culture supernatant obtained by adding Lamivudine to a culture of cells that have been infected with HBV and cultured to proliferate the HBV.
Fig. 6
   Fig. 6 is a graph, obtained in Example 4 with use of the present invention, which shows transitions in the numbers of copies of HCV RNA in cells infected with a patent's serum-derived HCV and cultured under respective conditions.
Fig. 7

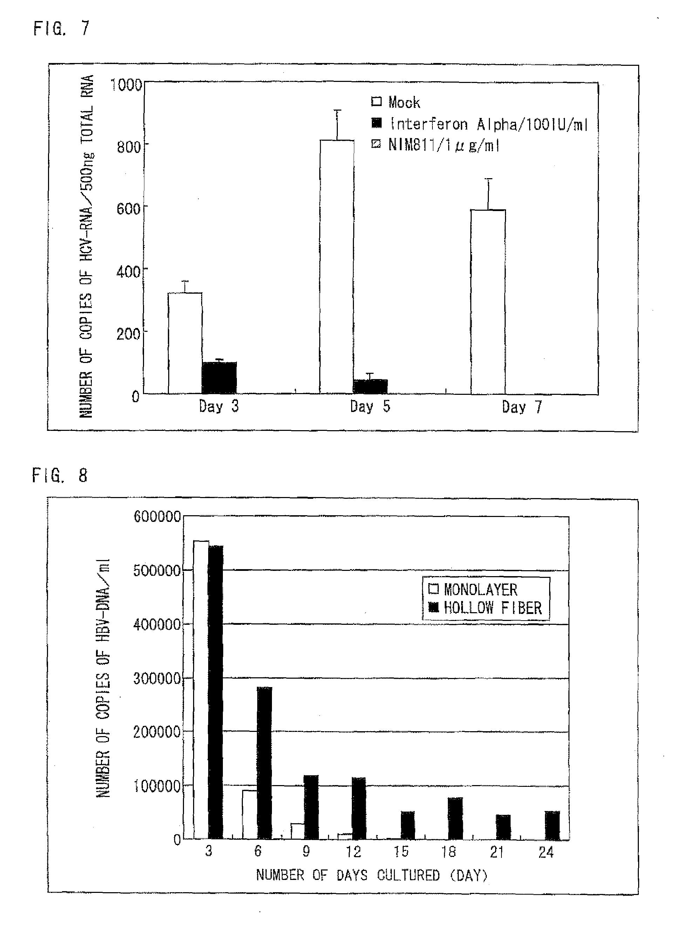

   Fig. 7 is a graph, obtained in Example 5 with use of the present invention, which shows transitions in the number of copies of HCV RNA in a culture supernatant obtained by adding interferon or NIM811 to a culture of cells infected with a patient's serum-derived HCV.
Fig. 8
   Fig. 8 is a graph, obtained in Example 6 with use of the present invention, which shows transitions in the number of copies of HBV DNA in a culture supernatant obtained from a culture of cells that have been infected with HBV and cultured under respective conditions to proliferate the HBV.
Fig. 9

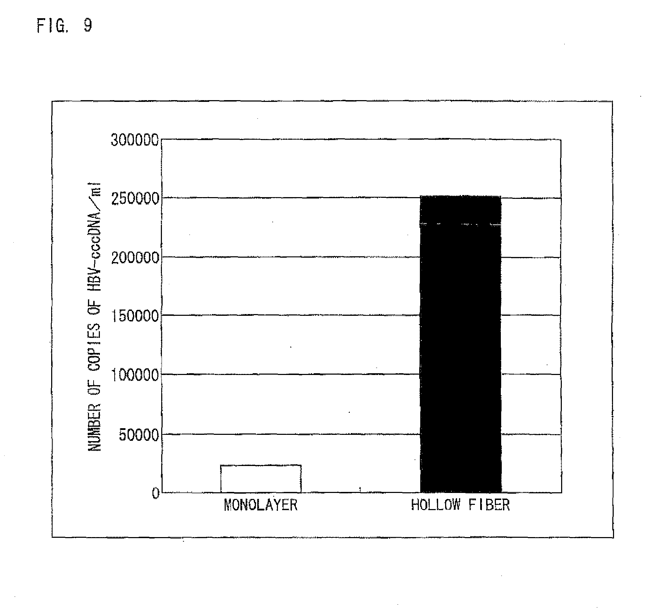

   Fig. 9 is a graph, obtained in Example 6 with use of the present invention, which shows transitions in the numbers of copies of HBV cccDNA in cells infected with HBV and cultured under respective conditions to proliferate the HBV.
Fig. 10

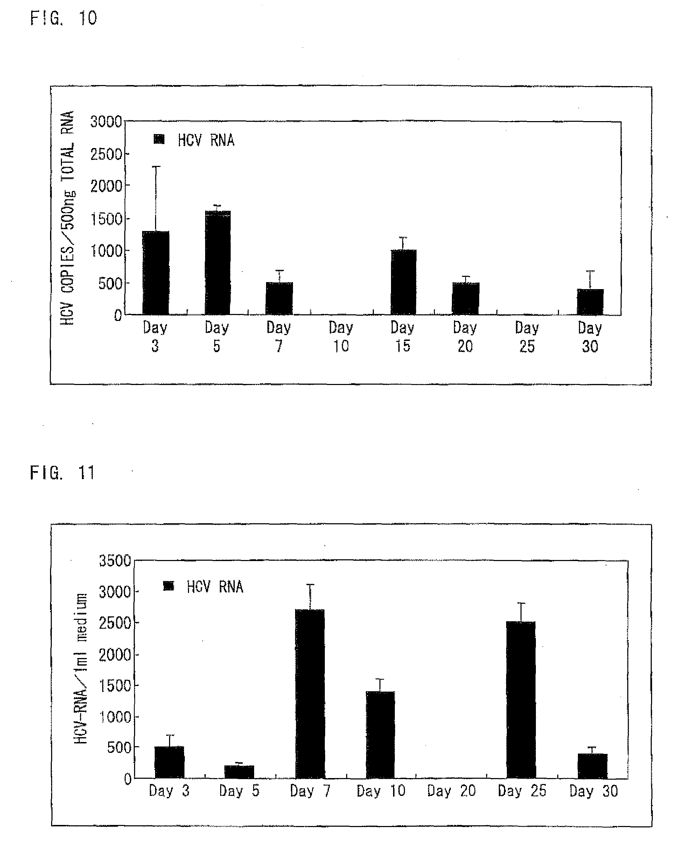

   Fig. 10 is a graph, obtained in Example 7 with use of the present invention, which shows transitions in the number of copies of HCV RNA in cultured cells infected with a patient's serum-derived HCV.
Fig. 11
   Fig. 11 is a graph, obtained in Example 7 with use of the present invention, which shows transitions in the number of copies of HCV RNA in a culture supernatant obtained from a culture of cells infected with a patient's serum-derived HCV.

### Description of Embodiments

A concrete embodiment of the present invention is described below. However, the present invention is not limited to this.

### (Method and Kit for Proliferating a Hepatitis Virus)

The present invention provides a method for proliferating a hepatitis virus. The present proliferation method includes the steps of: infecting, with a hepatitis virus, cells packed into a lumen of a hollow fiber made of a permeabilized membrane; and culturing the cells. Alternatively, the present proliferation method includes the steps of: infecting cells with a hepatitis virus; packing the cells into a lumen of a hollow fiber made of a permeabilized membrane; and culturing the cells. Each of the methods is characterized in culturing hepatitis virus-infected cells in a lumen of a hollow fiber.

### (Hollow Fiber)

The hollow fiber for use in the present invention can be suitably realized by a hollow fiber (i) made of a permeabilized membrane having pores that admit passage of culture liquid components such as water, salts, and protein while blocking passage of cells, (ii) including a lumen in which hepatitis virus-infected cells are retained, and (iii) having one end sealed and the other end opened. Thus, the hollow fiber for use in the present invention is not particularly limited in material as long as (i) it can retain cells in its lumen and (ii) it can be structured to admit passage of a solution or a low-molecular substance. Suitably usable examples of the material include a cellulosic material, a polyethylene, a polypropylene, and a polysulphone.

The hollow fiber is not particularly limited in size. However, it is preferable that the hollow fiber have an inner diameter of 20 µm to 1000 µm, or more preferably 50 µm to 500 µm. The hollow fiber is not particularly limited in membrane thickness, either. The membrane thickness of the hollow fiber may be set within a range that allows the hollow fiber to exhibit moderate strength as well as high permeability to a substance. For example, it is preferable that the hollow fiber have a membrane thickness of approximately 1.0 µm to 200 µm. In the case of use of a porous-membrane hollow fiber, it is preferable in view of cell culture that the porous-membrane hollow fiber have pores whose average diameter is so large that high matter exchange efficiency can be achieved. Therefore, a porous-membrane hollow fiber having pores whose average diameter is in a range of approximately 0.1 µm to 5 µm is preferably selected. However, the average diameter of the pores of the porous-membrane hollow fiber is not limited to such a range.

Further, such a hollow fiber may be used for the hepatitis virus proliferation method of the present invention in such a state as to be attached to an apparatus including (1) a cell suspension circulation tube, composed of an inflow circulation tube and an outflow circulation tube detachably jointed to each other, which has an end opened and (2) a hollow fiber fixation section provided at the other end of the cell suspension circulation tube. In this case, one or a plurality of hollow fibers, each having an end opened and the other end sealed, are attached to the apparatus so as to (1) be in communication with the cell suspension circulation tube via respective open ends and to (2) pass through the hollow fiber fixation section without causing a leak of the cell suspension.

Such a hollow fiber and an apparatus provided with a hollow fiber as used in the present invention can be suitably realized by their counterparts described in Japanese Patent Application Publication, Tokukai, No. 2005-110695. In the present invention, the descriptions in the above-referenced patent literature can be cited as appropriate.

### (Filling the Hollow Fiber with Cells)

The present proliferation method fills a lumen of the aforementioned hollow fiber with either cells that are to be infected with a hepatitis virus or cells infected with a hepatitis virus. It is preferable that the hollow fiber be finished with hydrophilation and sterilization before it is filled with the cells. The hollow fiber is subjected to hydrophilation and sterilization by conventional methods, respectively. Further, it is preferable that the lumen of the hollow fiber be filled with (i) a cell preservation solution such as a cell culture liquid medium, (ii) an adequate buffer solution, or (iii) the like before the hollow fiber is filled with the cells.

A cell suspension is prepared by suspending the cells, which are to be packed into the lumen of the hollow fiber, in (i) a cell culture liquid medium, (ii) a cell preservation solution, (iii) an adequate buffer solution, or (iv) the like. The cell suspension is then poured into the lumen of the hollow fiber via an opening of the hollow fiber. Preferably, the cell suspension may be prepared with use of (i) the cell culture liquid medium, (ii) the cell preservation solution, or (iii) the adequate buffer solution, with which the lumen of the hollow fiber has been filled in advance. Particularly, it is preferable that the cell suspension be prepared with use of a liquid medium having an identical composition.

The hollow fiber has the other end sealed. Therefore, while the cell suspension is poured into the lumen of the hollow fiber via the opening of the hollow fiber, a liquid component, such as the liquid medium or the like, contained in the cell suspension outflows via pores of the hollow fiber, and the cells are deposited in the lumen of the hollow fiber. The cells deposited in the lumen of the hollow fiber are not particularly limited in density. However, it is preferable that the cells be agglutinated by applying pressure or centrifugal force to the hollow fiber in which the cells have been packed and then deposited.

The agglutination of the cells packed into the lumen of the hollow fiber can be realized by making the inner pressure of the hollow fiber positive, by making the outer pressure of the hollow fiber negative, or by centrifuging the hollow fiber filled with the cells. The method for agglutinating the cells by applying centrifugal force to the hollow fiber can be suitably realized by a method disclosed in Japanese Patent Application Publication, Tokukai, No. 2004-166717, whereas the method for agglutinating the cells by applying pressure to the hollow fiber can be suitably realized by a method disclosed in Japanese Patent Application Publication, Tokukai, No. 2004-283010. In the present invention, the descriptions in the above-referenced patent literatures can be cited as appropriate.

### (Cells)

The cells for use in the present proliferation method are not particularly limited as long as they can be infected with a hepatitis virus. As such, hepatocytes or liver-derived cells can be suitably used. Particularly, it is preferable that human hepatocytes be used. Besides, it is most preferable that human liver-derived immortalized hepatocytes Hus-E/2 cells (FERM ABP-10908) be used, because use of the human liver-derived immortalized hepatocytes Hus-E/2 cells makes it possible to proliferate a hepatitis virus with particularly high efficiency. In this specification, the cells to be infected with a hepatitis virus are referred to sometimes as "cultured cells" or "host cells".

### (Hepatitis Virus)

In the present invention, the hepatitis virus with which the aforementioned cultured cells are infected and which is then allowed to keep the cultured cell infected or to proliferate is any of the following: hepatitis A virus; hepatitis B virus (HBV); hepatitis C virus (HCV); and hepatitis E virus. In particular, use of HBV or HCV brings about a remarkable effect. The hepatitis virus for use in the present invention is not particularly limited in form. As such, any of the following is suitably used, namely (i) a polynucleotide of hepatitis virus, (ii) a hepatitis virus particle, (iii) a serum containing a hepatitis virus particle, or (iv) a culture supernatant obtained by transiently introducing viral genes into the cultured cells.

The polynucleotide of the hepatitis virus for use in the present invention is a gnomic nucleic acid of hepatitis virus, and may exist in the form of DNA, RNA, or cDNA. The hepatitis virus particle, which may be referred to simply as a "hepatitis virus", has a genomic nucleic acid of a hepatitis virus contained in a virus capsid constituted by a constituent protein of the hepatitis virus particle, and has ability to infect cells. The hepatitis virus particle can be obtained from a cell into which hepatitis viral genes have been introduced to cause transient infection. The culture supernatant obtained from a culture of cells transiently infected with a hepatitis virus contains transiently proliferated hepatitis virus particles. The serum containing a hepatitis virus particle is obtained from blood collected from a hepatitis virus-infected person, any as such, contains a hepatitis virus of the hepatitis virus-infected person.

In particular, it is preferable that the hepatitis virus particle, the culture supernatant obtained from a culture of cells transiently infected with a hepatitis virus, or the serum containing a hepatitis virus particle be used, because use of one of them makes it easy to infect the cells by contact. In the present invention, the serum containing a hepatitis virus particle can be particularly suitably used.

### (Hepatitis Virus-infected Cells)

According to the present proliferation method, hepatitis virus-infected cells are cultured in a lumen of a hollow fiber. It is possible that the cells are infected with a hepatitis virus before or after being packed into the lumen of the hollow fiber.

The method for infecting the cells with a hepatitis virus is not particularly limited, and as such, can be suitably realized by either (i) a method for introducing hepatitis viral genes into cells by electroporation or the like or (ii) a method for infecting cells by bringing the cells into contact with a hepatitis virus particle for a certain period of time. The method for infecting cells by contact with a hepatitis virus particle is preferred because the method makes it possible to easily obtain virus-infected cells. It is also possible to obtain virus-infected cells by contact with a culture supernatant or a serum containing a hepatitis virus particle. In the present invention, particularly, virus-infected cells obtained through infection by contact with a serum obtained from a hepatitis virus-infected person is suitably used.

In a case where cells are infected with a hepatitis virus by contact before being packed into a lumen of a hollow fiber, the cells are dispersed in a liquid medium or the like and are in a suspended state, and as such, exhibit high infection efficiency with increased opportunities to make contact with the hepatitis virus. On the other hand, in a case where cells are infected with a hepatitis virus by contact after being packed into a lumen of a hollow fiber and being agglutinated by applying pressure or centrifugal force to the hollow fiber, the hepatitis virus exhibits such high infectious capacity as to stably infect the cells. Use of a hollow fiber made of a permeabilized membrane having pores whose average diameter is sufficiently larger than a hepatitis virus particle allows a hepatitis virus to reach a surface of the respective cells via the pores in the membrane constituting the hollow fiber.

The infection of cells with a hepatitis virus here means a state in which the hepatitis virus has invaded the cells and hepatitis virus particles are being stably proliferated. Thus, the infection with the hepatitis virus can be confirmed by (i) determining the amount of a nucleic acid of the hepatitis virus in the cells or in a culture supernatant obtained from a culture of the cells or (ii) determining the amount of a constituent protein of the hepatitis virus in the cells or in a culture supernatant obtained from a culture of the cells. Particularly, infection with hepatitis B virus can be confirmed by determining the amount of cccDNA (covalently closed circular DNA: replicative intermediate DNA) contained in cells.

### (Culture)

A hollow fiber for culture in the present proliferation method is a hollow fiber for culturing hepatitis virus-infected cells. As described above, such a hollow fiber can be manufactured by either (i) infecting, with a hepatitis virus, cells packed into a lumen of the hollow fiber or (ii) infecting cells with a hepatitis virus and then packing them into a lumen of the hollow fiber.

The hepatitis virus-infected cells thus contained in the hollow fiber are cultured by a conventional method. For example, the hollow fiber containing the hepatitis virus-infected cells is placed in a culture vessel, and then the hepatitis virus-infected cells are cultured at 37°C with 5% CO₂. In this case, the cells in the lumen of the hollow fiber can be efficiently supplied with nutrients and oxygen by culturing them while shaking the culture vessel by a shaker or the like.

By culturing the hepatitis virus-infected cells as described above, the hepatitis virus is proliferated in the cultured cells. Proliferation of the hepatitis virus in the cells can be confirmed by (i) determining the amount of a nucleic acid of the hepatitis virus in the cells or in a culture supernatant obtained from a culture of the cells or (ii) determining the amount of a constituent protein of the hepatitis virus particle in the cells or in a culture supernatant obtained from a culture of the cells. Particularly, proliferation of hepatitis B virus can be confirmed by determining the amount of cccDNA (covalently closed circular DNA: replicative intermediate DNA) in cells.

The present proliferation method makes it possible to proliferate a hepatitis virus *in vitro* by culturing hepatitis virus-infected cells with use of the hallow fiber. Further, the present invention allows the hepatitis virus to cause infection and proliferate in the cells for a long period of time. Furthermore, the present invention makes it possible to easily and efficiently proliferate the hepatitis virus with use of cells naturally infected with the hepatitis virus by contact, instead of use of cells forcibly infected with the hepatitis virus through introduction of genes of the hepatitis virus. Moreover, the present invention uses a hollow fiber of a simple structure, and as such, allows hepatitis virus infection and proliferation through a simple operation with use of a simple apparatus, without requiring a huge special apparatus.

As described above, the present invention makes it possible to proliferate a hepatitis virus with high efficiency. This makes it possible to suitably carry out (i) evaluation of a drug that inhibits hepatitis virus infection or proliferation, (ii) evaluation of a drug for treating a hepatitis virus-infected person, (iii) treatment of the hepatitis virus-infected person based on the evaluation of the drug, and (iv) the like with use of the hepatitis virus proliferated by the present invention. Furthermore, the present invention makes it possible to proliferate a hepatitis virus with high efficiency also in cells infected with use of a serum obtained from a hepatitis virus-infected person. The hepatitis virus thus proliferated can be applied to tailor-made diagnosis or treatment for individually diagnosing or treating the hepatitis virus-infected person. Particularly, such hepatitis virus is useful in evaluating a therapeutic drug individually for that hepatitis virus-infected person.

The present invention further provides a kit for proliferating a hepatitis virus. The present kit includes at least a hollow fiber, made of a permeabilized membrane, whose lumen has been filled with hepatitis virus-infectable cells. As such, the kit according to the present invention for proliferating a hepatitis virus should include at least the hollow fiber for culturing hepatitis virus-infected cells. The hepatitis virus can be proliferated by culturing the hepatitis virus-infected cells in the lumen of the hollow fiber. Alternatively, the present kit may include cells infected with a hepatitis virus.

### (Method for Producing a Hepatitis Vaccine)

The present invention further provides a method for producing a hepatitis vaccine with use of a culture of hepatitis virus-infected cells, a hepatitis virus particle, or a constituent protein of a hepatitis virus (hereinafter referred to as "hepatitis virus particle or the like") each obtained by the above proliferation method. The culture of hepatitis virus-infected cells includes cells infected with a hepatitis virus, a culture supernatant obtained by culturing the cells, or the like. The present production method makes it possible to produce a hepatitis vaccine by a conventional method with use of (i) the hepatitis virus particle or the like or (ii) a part of the hepatitis virus particle or the like as an antigen. For example, the hepatitis vaccine can be produced directly from the hepatitis virus particle or the like. Also, the hepatitis vaccine can be produced from a hepatitis virus or the like which has been attenuated or inactivated by a conventional method.

The hepatitis vaccine produced by the present invention is made administerable by dissolving or suspending it in a liquid. The hepatitis virus thus made administrable may be arranged in a form suitable for administration or may be put with a medically allowable component. The route of administration, the dosage, the number of doses, or the like of the hepatitis vaccine thus made administerable should be determined in the same manner as those of a conventional hepatitis vaccine or other types of vaccine are determined.

The present invention makes it possible to proliferate a hepatitis virus *in vitro* for a long period of time through a simple operation with use of a simple apparatus, and to efficiently produce a hepatitis vaccine with use of a hepatitis virus particle or the like so obtained. When administered to a living organism uninfected with the hepatitis virus, the hepatitis vaccine produced by the present invention induces an immune response to the hepatitis virus within the living organism. This makes it possible to prevent the living organism from being infected with the hepatitis virus. Alternatively, when administered to a living organism infected with the hepatitis virus, the hepatitis vaccine produced by the present invention induces a strong immune response to the hepatitis virus within the living organism. This makes it possible to use the hepatitis vaccine in treatment that removes the hepatitis virus.

### (Method and Kit for Screening a Drug)

Furthermore, the present invention provides a method for evaluating the effect of a drug on a hepatitis virus with use of the hepatitis virus-infected cells cultured in a lumen of a hollow fiber made of a permeabilized membrane. Specifically, the present invention provides a method for screening a drug that inhibits hepatitis virus proliferation or hepatitis virus protein expression, the method including the step of culturing, together with a drug candidate substance that is to be evaluated, hepatitis virus-infected cells packed into a lumen of a hollow fiber made of a permeabilized membrane. Also, the present invention provides a method for screening a drug that inhibits hepatitis virus infection, the method including the step of culturing, together with a hepatitis virus and a drug candidate substance that is to be evaluated, hepatitis virus-infectable cells packed into a lumen of a hollow fiber made of a permeabilized membrane.

In the culturing step, in a case where there is a change in a state of hepatitis virus infection, a state of hepatitis virus proliferation, or an expression level of hepatitis virus protein in the presence or absence of the drug candidate substance to be evaluated (i.e., in a case where hepatitis virus infection, hepatitis virus proliferation, or hepatitis virus protein expression is inhibited in the presence of the drug candidate substance to be evaluated), the drug candidate substance can be judged as a drug that inhibits hepatitis virus infection, hepatitis virus proliferation, or hepatitis virus protein expression.

The effect of the drug candidate substance on the state of hepatitis virus infection, the state of hepatitis virus proliferation, or the like can be evaluated by (i) adding the drug candidate substance to be evaluated into a culture fluid for culturing hepatitis virus-infected cells or a culture fluid for culturing hepatitis virus-infectable cells together with the hepatitis virus, (ii) culturing the hepatitis virus-infected cells or the hepatitis virus-infectable cells, and (iii) quantitatively and qualitatively analyzing the hepatitis virus nucleic acid, the constituent protein of the hepatitis virus particle, or the like contained in a culture supernatant or in the cells. This makes it possible not only to screen a drug that inhibits hepatitis virus infection, hepatitis virus proliferation, or hepatitis virus protein expression, but also to evaluate the resistance of the hepatitis virus to the drug or to understand the mechanism of the hepatitis virus.

The present invention allows the hepatitis virus to cause infection and proliferate in the cultured cells for a long period of time with high efficiency. Use of such hepatitis virus-infected cells makes it possible to screen a drug that inhibits hepatitis virus infection, hepatitis virus proliferation, or hepatitis virus protein expression. This makes it possible to evaluate various drugs with high efficiency. Further, the present invention makes it possible to proliferate a hepatitis virus with high efficiency also in cells infected with the hepatitis virus by contact with use of a serum derived from a hepatitis virus-infected person. This makes it possible to evaluate a drug candidate substance individually for each hepatitis virus-infected person.

The present invention further provides a kit for screening a drug that inhibit hepatitis virus infection, hepatitis virus proliferation, or hepatitis virus protein expression. The present kit includes at least a hollow fiber, made of a permeabilized membrane, whose lumen has been filled with hepatitis virus-infectable cells. As such, the present kit should include at least the hollow fiber for culturing hepatitis virus-infected cells.

A hepatitis virus can be proliferated with high efficiency by infecting, with the hepatitis virus, the cells packed into the lumen of the hollow fiber and culturing the cells, and use of such hepatitis virus-infected cells makes it possible to evaluate various drug candidate substances with high efficiency. In this case, use of hepatitis virus-infected cells infected with a hepatitis virus by contact with use of a serum derived from a hepatitis virus-infected person makes it possible to evaluate a drug candidate substance individually for that hepatitis virus-infected person. Alternatively, the present kit may include a hollow fiber whose lumen has been filled with hepatitis virus-infected cells. This makes it possible to evaluate various drugs efficiently with use of cells cultured by using the hollow fiber.

### (Method and Kit for Screening a Therapeutic Drug)

The screening method described above is a method for screening a drug that inhibits hepatitis virus infection or proliferation. However, the present invention is not limited to this. That is, the present invention also provides a method for screening a drug for treating a hepatitis virus-infected person. The present method includes a screening step identical to the method for screening a drug that inhibits hepatitis virus infection, hepatitis virus proliferation, or hepatitis virus protein expression. As such, the present method includes either the step of culturing, together with a drug candidate substance that is to be evaluated, cells packed into a lumen of a hollow fiber made of a permeabilized membrane and infected with a hepatitis virus, or the step of culturing, together with a hepatitis virus and a drug candidate substance that is to be evaluated, hepatitis virus-infectable cells packed into a lumen of a hollow fiber made of a permeabilized membrane. The present method allows the hepatitis virus to cause infection and proliferate in the cultured cells for a long period of time with high efficiency, and since use of such hepatitis virus-infected cells makes it possible to screen a drug for treating a hepatitis virus-infected person, the present method makes it possible to efficiently screen a drug suitable to treating the hepatitis virus-infected person.

In particular, with the present method, use of cells infected with a hepatitis virus by contact with use of a serum derived from a hepatitis virus-infected person makes it possible to individually screen a drug suitable to treating that hepatitis virus-infected person. As such, the present method can be suitably applied to tailor-made diagnosis or treatment for individually diagnosing or treating a hepatitis virus-infected person.

Further, the present invention provides a kit for screening a drug for treating a hepatitis virus-infected person. The present kit is arranged in the same manner as the kit for screening a drug that inhibits hepatitis virus infection, hepatitis virus proliferation, or hepatitis virus protein expression. Use of the present kit makes it possible to proliferate a hepatitis virus with high efficiency, and use of such hepatitis virus-infected cells makes it possible to efficiently screen a drug for treating a hepatitis virus-infected person. Particularly, use of hepatitis virus-infected cells infected with a hepatitis virus by contact with use of a serum derived from a hepatitis virus-infected person makes it possible to screen a drug individually for that hepatitis virus-infected person.

### (Method and Kit for Finding a Combination of Drugs Most Suitable for Treatment)

Furthermore, the present invention provides a method for finding a combination of drugs most suitable for treating a hepatitis virus-infected person. The present method includes a screening step identical to the method for screening a drug that inhibits hepatitis virus infection, hepatitis virus proliferation, or hepatitis virus protein expression. As such, the present method includes either the step of culturing, together with a plurality of drug candidate substances that are to be evaluated, cells packed into a lumen of a hollow fiber made of a permeabilized membrane and infected with a hepatitis virus, or the step of culturing, together with a hepatitis virus and a drug candidate substance that is to be evaluated, hepatitis virus-infectable cells packed into a lumen of a hollow fiber made of a permeabilized membrane. The present method allows the hepatitis virus to cause infection and proliferate in the cultured cells for a long period of time with high efficiency, and since use of such hepatitis virus-infected cells makes it possible to find a combination of drugs most suitable for treating a hepatitis virus-infected person, the present method makes it possible to efficiently screen a combination of drugs suitable to treating the hepatitis virus-infected person. This makes it possible to establish customized treatment.

In particular, with the present method, use of culture cells infected with a hepatitis virus by contact with use of a serum derived from a hepatitis virus-infected person makes it possible to individually find a combination of drugs suitable to treating that hepatitis virus-infected person. As such, the present method can be suitably applied to tailor-made diagnosis or treatment for individually diagnosing or treating a hepatitis virus-infected person.

Further, the present invention provides a kit for finding a combination of drugs most suitable for treating a hepatitis virus-infected person. The present kit is arranged in the same manner as the kit for screening a drug that inhibits hepatitis virus infection, hepatitis virus proliferation, or hepatitis virus protein expression. Use of the present kit makes it possible to proliferate a hepatitis virus with high efficiency, and use of such hepatitis virus-infected cells makes it possible to efficiently find a combination of drugs most suitable to treating a hepatitis virus person. Particularly, use of hepatitis virus-infected cells infected by contact with use of a serum derived from a hepatitis virus-infected person makes it possible to find a combination of drugs most suitable for individually treating that hepatitis virus-infected person.

### (Diagnostic or Examination Method)

Furthermore, the present invention provides a diagnostic or examination method for treating a hepatitis virus-infected person. The diagnostic or examination method according to the present invention includes the step of culturing, together with a drug that is to be evaluated, hepatitis virus-infected cells packed into a lumen of a hollow fiber made of a permeabilized membrane. The culturing step further includes the steps of: determining a drug for treatment of a hepatitis virus-infected person by drug evaluation and (ii) administering the drug thus determined. Specifically, in the culturing step, in a case where there is a change in a state of hepatitis virus infection or a state of hepatitis virus proliferation in the presence or absence of the drug to be evaluated (i.e., in a case where hepatitis virus infection or hepatitis virus proliferation is inhibited in the presence of the drug to be evaluated), the drug can be judged as a drug for treatment of the hepatitis virus-infected person. The drug thus evaluated is administered as a drug for treatment of the hepatitis virus-infected person, whereby the hepatitis virus-infected person is treated.

The effect of the drug on the state of hepatitis virus infection or the state of hepatitis virus proliferation can be evaluated by (i) adding the drug to be evaluated into a culture fluid for culturing hepatitis virus-infected cells or a culture fluid for culturing hepatitis virus-infectable cells together with the hepatitis virus, (ii) culturing the hepatitis virus-infected cells or the hepatitis virus-infectable cells, (iii) quantitatively and qualitatively analyzing the hepatitis virus nucleic acid, the constituent protein of the hepatitis virus particle, or the like contained in a culture supernatant or in the cells. This makes it possible to predict the effect of the drug on a hepatitis virus-infected person.

The present invention allows the hepatitis virus to cause infection and proliferate in the cultured cells for a long period of time with high efficiency, and since use of such hepatitis virus-infected cells makes it possible to screen a drug that inhibits hepatitis virus infection, hepatitis virus proliferation, or hepatitis virus protein expression, the present invention makes it possible to efficiently evaluate the effects of various drugs on hepatitis virus-infected persons. Further, the present invention makes it possible to proliferate a hepatitis virus with high efficiency also in cells infected with the hepatitis virus by contact with use of a serum derived from a hepatitis virus-infected person. This makes it possible to evaluate a drug individually for each hepatitis virus-infected person. As such, the present diagnostic method can be suitably applied to tailor-made diagnosis or treatment for individually diagnosing or treating a hepatitis virus-infected person.

The present invention will be detailed below with reference to examples of experiment. However, the present invention is not limited to these examples.

### (Example 1)

A cell suspension was prepared by melting frozen human hepatocytes (Xenotech, Code: H1000. H15, lot. 428) in a culture fluid and then suspending the human hepatocytes in the culture fluid at a cell concentration of approximately 2.0 × 10⁶ cell/ml, Into 1 ml of the cell suspension, 100 µl of an HBV-infected person's serum containing a high concentration of HVB were added. Then, the cell suspension was left at rest for 60 minutes at 37°C with 5% CO₂ and 95% atmosphere. A suspension of human hepatocytes was prepared in the same manner as described above. Into the suspension, a virus particle-containing culture supernatant obtained by (i) transfecting an HBV C-22 strain into Huh7 cells instead of the HBV-infected person's serum, and (ii) transiently proliferating the HBV C-22 strain was added. Then, the suspension was left at rest for 60 minutes 37°C with 5% CO₂ and 95% atmosphere. After that, each of the suspensions of human hepatocytes was poured into a lumen of a hollow fiber via a cellulose triacetate hollow fiber module (Toyobo Co., Ltd.; Code: TMHFK-001; having an inner diameter of 285 µm, an outer diameter of 387 µm, and a pore diameter of 0.2 µm). The cells were packed into the lumen of the hollow fiber by applying a centrifugal force of 60G for 90 seconds. That portion of the hollow fiber whose lumen had been filled with the cells was cut out and moved to a 12-well cell culture plate, and then put into a commercially-available human hepatocellular serum-free culture fluid (Toyobo Co., Ltd.; Code: TMHHM-001) suitable to culturing human hepatocytes. Then, the cells were shake-cultured at 37°C with 5% CO₂ and 95% atmosphere.

Two days after the start of culture and infection, the hollow fiber was washed with a culture fluid four times. After that, the culture lasted until the 35th days with the culture fluid changed every two days. DNA in the culture fluid was extracted from the cell culture supernatant with use of a commercially-available DNA extraction kit (QIAamp blood kit, QIAGEN), and then assayed over time for HBV DNA by a Real-time PCR. Also, intracellular DNA was extracted from a cytolytic fluid prepared by collecting and homogenizing the cells on the 35th day of the culture, and then assayed for HBV-specific cccDNA (replicative intermediate DNA) by the Real-time PCR.

As a result, it was confirmed between the 10th to 35th days of culture that culture supernatant of the cells infected with the HBV-infected person's serum contained HBV DNA corresponding to HBV particles in a range of 7.0 × 10³ to 5.0 × 10⁴ copies/ml and the culture supernatant of the cells infected with a virus fluid obtained from the culture fluid contained HBV DNA corresponding to HBV particles in a range of 6.5 × 10⁶ to 1.7 × 10⁶ copies/ml.

Also, it was confirmed on the 35th day of the culture that the cells infected with the serum contained cccDNA corresponding to 7.3 × 10² copies and the cells infected with the virus fluid obtained from the culture fluid contained cccDNA corresponding to 4.0 × 10⁸ copies. This showed that HBV caused infection and proliferated in the cells for a long period of time.

### (Example 2)

A cell suspension was prepared by melting frozen human hepatocytes (Xenotech, Code: H1000. H15, lot. 428) in a culture fluid and then suspending the human hepatocytes in the culture fluid at a cell concentration of approximately 2.0 × 10⁶ cells/ml. After this, 1 ml of the suspension of human hepatocytes was poured into a lumen of a hollow fiber via a cellulose triacetate hollow fiber module (Toyobo Co., Ltd.; Code: TMHFK-001; having an inner diameter of 285 µm, an outer diameter of 387 µm, and a pore diameter of 0.2 µm). The cells were packed into the lumen of the hollow fiber by applying a centrifugal force of 60G for 90 seconds. That portion of the hollow fiber whose lumen had been filled with the cells was cut out and moved to a 12-well cell culture plate, and then put into a commercially-available human hepatocellular serum-free culture fluid (Toyobo Co., Ltd.; Code: TMHHM-001) suitable to culturing human hepatocytes. Into this, a virus particle-containing culture supernatant obtained by (i) transfecting an HBV C-22 strain into Fuh7 cells, and (ii) transiently proliferating the HBV C-22 strain was added. Then, the cells were shake-cultured at 37°C with 5% CO₂ and 95% atmosphere.

Two days after the start of culture and infection, the hollow fiber was washed with a culture fluid four times. After that, the culture lasted until the 35th days with the culture fluid changed every two days. DNA in the culture fluid was extracted from the cell culture supernatant with use of a commercially-available DNA extraction kit (QIAamp blood kit, QIAGEN), and then assayed over time for HBV DNA by a Real-time PCR. Also, intracellular DNA was extracted from a cytolytic fluid prepared by collecting and homogenizing the cells on the 35th day of the culture, and then assayed for HBV-specific cccDNA (replicative intermediate DNA) by the Real-time PCR.

As a result, it was confirmed between the 10th to 35th days of the culture that the cell culture supernatant contained HBV DNA corresponding to HBV particles in a range of 6.0 × 10⁵ to 5.0 × 10⁷ copies/ml. Also, it was confirmed on the 35th day of the culture that the cells infected with the cell culture supernatant contained cccDNA corresponding to 4.1 × 10⁷ copies. This showed that HBV caused infection and proliferated in the cells for a long period of time.

### (Example 3)

A cell suspension was prepared by melting frozen human hepatocytes (Xenotech, Code: H1000. H15, lot. 428) in a culture fluid and suspending the human hepatocytes in the culture fluid at a cell concentration of approximately 2.0 × 10⁶ cells/ml. After that, the cell suspension of human hepatocytes was poured into a lumen of a hollow fiber of a cellulose triacetate hollow fiber module (Toyobo Co., Ltd.; Code: TMHFK-001; having an inner diameter of 285 µm, an outer diameter of 387 µm, and a pore diameter of 0.2 µm). The cells were packed into the lumen of the hollow fiber by applying a centrifugal force of 60 G for 90 seconds. That portion of the hollow fiber whose lumen has been filled with the cells was cut out and moved to a 12-well cell culture plate, and then put into a commercially-available human hepatocellular serum-free culture fluid (Toyobo Co., Ltd.; Code: TMHHM-001) suitable to culturing human hepatocytes. Then, the cells were subjected to shake-culture at 37°C with 5% CO₂ and 95% atmosphere. The culture medium was changed every other day. On the 8th day of culture, the cells were infected with HBV by adding an HBV fluid into the culture fluid. The HBV fluid was prepared by condensing a culture supernatant obtained by (i) transfecting an HBV Ae strain into Huh7 cells, (ii) transiently infecting the Huh7 cells with the HBV Ae strain, and (iii) culturing the Huh7 cells. Simultaneously with the infection, lamivudine, which is clinically applied as a therapeutic drug for treating chronic hepatitis B disease, was added to the cell culture medium so as to have a final concentration of 0.2 mM or 0.1 mM. The cells were then cultured. The culture supernatant, to which lamivudine had been added, was assayed over time for an HBV DNA amount by the Real-time PCR.

As a result, in comparison with the control group, the lamivudine-added group exhibited a remarkably suppressed increase in HBV DNA contained in the cell culture supernatant, whereby the antiviral effect of the drug was confirmed.

### (Example 4)

Human liver-derived immortalized hepatocytes HuS-E/2 cells were used in the experiment. The HuS-E/2 cells were established in the following manner. It should be noted that the HuS-E/2 cells have been deposited in the International Patent Organism Depositary of the National Institute of Advanced Industrial Science and Technology on September 7, 2007 (Depository No. FERM ABP-10908).

A human telomerase reverse transcriptase gene (hTERT gene) and a human papillomavirus E6E7 gene (HPV18/E6E7), which had both been inserted into a plasmi, were transfected in the usual manner into normal human hepatocytes separated from human hepatic tissue. After the transfection of the genes, those human hepatocytes which had highly expressed hepatocellular function were selected from a cell colony which had been immortalized by acquiring proliferation potency. Thus, HuS-E/2 clones were obtained.

### (Example 4-1: Infection and Culture by a Monolayer Culture Method)

HuS-E/2 cells were dispersed in a 12-swell collagen-coated plate so as to have a density of 1 × 10⁵ cells/well. After 24 hours, the culture fluid was replaced with 1 ml of a human hepatocellular serum-free culture fluid containing HCV with 2 × 10⁵ genome copies/ml (Toyobo Co., Ltd.; Code: TMHHM-001). The HCV had been derived from a serum of an HCV-infected person. After another 24 hours, the culture fluid was removed, and the HuS-E/2 cells were washed with PBS three times. Then, a new culture fluid was added. After that, the culture medium was changed every two days. The HuS-E/2 cells were collected 3, 5, and 7 days after infection with serum-derived HCV, and their total RNA was collected with use of a Sepasol I (Nacalai Tesque, Inc). The total RNA was assayed for HCV RNA by a PCR method.

### (Example 4-2: Infection by a Monolayer Culture Method and Culture in a Hollow Fiber Thereafter)

HuS-E/2 cells were cultured under the same conditions as in Example 4-1, and then were similarly infected with serum-derived HCV. After 48 hours, the HuS-E/2 cells were collected from six wells of a 12-well collagen-coated plate with use of trypsin, and then were packed into lumens of cellulose triacetate hollow fiber modules (Toyobo Co., Ltd.; Code: TMHFK-001), respectively, by applying a centrifugal force of 80 G for 90 seconds. Each of the cellulose triacetate hollow fiber modules was subjected to shake-culture in a well of the 12-well plate. The culture fluid was replaced with a new culture fluid every two days. Total RNA of the HuS-E/2 cells in the cellulose triacetate hollow fiber module was collected in the usual manner after the same numbers of days as in Example 4-1. The total RNA was assayed for HCV RNA in the same manner as in Example 4-1.

### (Example 4-3: Infection and Culture by a Hollow Fiber Culture Method)

HuS-E/2 cells with a density of 1 × 10⁵ cells/well were packed into a lumen of a hollow fiber of a cellulose triacetate hollow fiber module by applying a centrifugal force of 80 G for 90 seconds, and then were shake-cultured with use of a 12-well plate. After 24 hours, the culture fluid was replaced with 0.8 ml of a culture fluid containing serum-derived HCV with 2 × 10⁶ genome copies/ml. After another 24 hours, the culture fluid was removed, and the HuS-E/2 cells were washed with PBS three times. Then, a new culture fluid was added. The culture fluid was changed every two days. Total RNA of the HuS-E/2 cells in the cellulose triacetate hollow fiber module was collected in the usual manner after the same numbers of days as in Example 4-1. The total RNA was assayed for HCV RNA in the same manner as in Example 4-1.

As a result, it was shown that, even in those HuS-E/2 cells cultured and infected by the monolayer culture method, the serum-derived HCV transiently caused infection and proliferated at a very low level. However, the level was so low, for example, that it was impossible to judge the effectiveness of a drug with use of the culture system. In contrast, those HuS-E/2 cells packed into and cultured in a hollow fiber exhibited a remarkably high viral infectivity. Furthermore, those HuS-E/2 cells infected with the virus in monolayer culture, digested by trypsin, collected, and packed into a lumen of a hollow fiber exhibited substantially the same virus infectivity as those HuS-E/2 cells infected and cultured by the monolayer culture method.

The results of Example 4 showed that three-dimensional culture of cells packed into a hollow fiber makes it possible to obtain a high viral infectivity by infecting the cultured cells with a virus derived from a serum of an HCV-infected person and proliferating the virus in the cultured cells. Example 4 also suggested that it is important that the cells be three-dimensionally structured in a hollow fiber when infected with HCV. It was inferred from this, for example, that a hepatocyte expresses its original function when polarized toward a vascular side or a biliary side, and as a result, is infected with HCV in a similar state as *in vivo.*

### (Example 5)

HuS-E/2 cells with a density of 1 × 10⁵ cells/well were packed into a lumen of a hollow fiber of a cellulose triacetate hollow fiber module (Toyobo Co., Ltd.; Code: TMHFK-001) by applying a centrifugal force of 80 G for 90 seconds, and then were shake-cultured with use of a 12-well plate. After 24 hours, the culture fluid was replaced with 0.8 ml of a culture fluid, prepared with use of human hepatocellular serum-free culture fluid (Toyobo Co., Ltd.; Code: TMHHM-001), which contained serum-derived HCV with 2 × 10⁵ genome copies/ml. After 24 hours of infection, the culture fluid was removed, and the HuS-E/2 cells were washed with PBS three times. Then, three types of new culture fluid were separately added, namely a culture fluid containing INF alpha (1000 IU/ml), a culture fluid containing NIM811 (1 µg/ml), and a culture fluid containing neither of them. Each of the culture fluids was replaced with a new one every two days. The HuS-E/2 cells were collected after 3, 5, and 7 days of infection with serum-derived HCV, and their total RNA was collected with use of a Sepasol I (Nacalai Tesque, Inc). The total RNA was assayed for HCV RNA by a PCR method.

As a result, it was confirmed that addition of an anti-HBV drug such as INF alpha or NIM811 to a cell culture medium remarkably inhibits patient's serum-derived HCV from proliferating in the cells. The group given no drug exhibited a high viral infectivity. It should be noted that NIM 811 is a cyclosporine derivative reported to inhibit HCV genome replication.

### (Example 6)

A cell suspension was prepared so as to have an HuS-E/2 cell concentration of approximately 2.0 × 10⁶ cells/ml. Then, the HuS-E/2 cells were infected with HBV at 37°C for one hour by adding an HBV fluid into the cell suspension. The HBV fluid was prepared by condensing a culture supernatant obtained by (i) transfecting an HBV C-AT strain into Huh7 cells, (ii) transiently infecting the Huh7 cells with the HBV C-AT strain, and (iii) culturing the Huh7 cells.

### (Example 6-1)

HuS-E/2 cells infected with HBV were dispersed in a 12-well collagen-coated plate so as to have a density of 1.5 × 10⁵ cells/well. Then, the HuS-E/2 cells were cultured with use of a human hepatocellular serum-free culture fluid (Toyobo Co., Ltd.; Code: TMHHM-001) with the culture medium changed every three days. The culture supernatant was assayed over time for HBV DNA by a Real-time PCR. Also, intracellular DNA was extracted from a cytolytic fluid prepared by collecting and homogenizing the cells on the 24th day of the culture, and then assayed for HBV-specific cccDNA (replicative intermediate DNA) by a Real-time PCR.

### (Example 6-2)

HBV-infected HuS-E/2 cells with a density of 1.5 × 10⁵ were packed into a lumen of a hollow fiber of a cellulose triacetate hollow fiber module (Toyobo Co., Ltd.; Code: TMHFK-001) by applying a centrifugal force of 80 G for 90 seconds. After being moved to a 12-well cell culture plate, the HuS-E/2 cells were shake-cultured with use of the same culture fluid as in Example 6-1. With the culture medium changed every three days, the culture supernatant was assayed over time for HBV DNA by the real-time PCR. Also, intracellular DNA was extracted from a cytolytic fluid prepared by collecting and homogenizing the cells on the 24th day of the culture, and then assayed for HBV-specific cccDNA (replicative intermediate DNA) by the Real-time PCR.

As a result, the hollow-fiber culture maintained a higher HBV infectivity titer for a longer period of time than the monolayer culture. Besides, the hollow-fiber culture resulted in a remarkably larger number of copies of cccDNA than the monolayer culture.

### (Example 7)

HuS-E/2 cells with a density of 1 × 10⁵ cells/well were packed into a lumen of a hollow fiber of a cellulose triacetate hollow fiber module by applying a centrifugal force of 80 G for 90 seconds, and then were shake-cultured with use of a 12-well plate. After 24 hours, the culture fluid was replaced with 0.8 ml of a culture fluid containing serum-derived HCV with 2 × 10⁵ genome copies/ml. After another 24 hours, the culture fluid containing the above serum-derived HCV was removed, and the HuS-E/2 cells were washed with PBS three times. Then, a new culture fluid was added. After that, the culture lasted until the 30th day with the culture fluid changed every two days. Meanwhile, total DNA was collected over time from each of the HuS-E/2 cells and the cell culture supernatant in the module in the usual manner, and then checked for transitions in the number of copies of HCV RNA.

As the result of examining the transitions in the number of copies of HCV RNA, it was found that the HCV infection lasted as long as 30 days or more through repetition of increases and decreases in the number of copies of HCV RNA. It was also found that the HCV RNA was present in the culture supernatant for 30 days or more.

As a result, it was confirmed that cells packed into a hollow fiber and infected with a virus derived from a serum of an HCV-infected person kept a high level of infection for a long period of time.

As described above, the present invention makes it possible to efficiently proliferate a hepatitis virus *in vitro* with long-term stability by culturing hepatitis virus-infected cells packed into a lumen of a hollow fiber made of a permeabilized membrane. Furthermore, the present invention makes it possible to evaluate the effect of a drug on a hepatitis virus with use of hepatitis virus-infected cells cultured as described above.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### Industrial Applicability

The present invention makes it possible to efficiently proliferate a hepatitis virus *in vitro* at a high infectivity titer with long-term stability by infecting, with the hepatitis virus, cultured cells packed into a lumen of a hollow fiber made of a permeabilized membrane. Furthermore, the present invention makes it possible to carry out a drug evaluation method for evaluating the effect of a drug on a hepatitis virus with use of hepatitis virus-infected cells cultured as described above.

There has been no culture system that makes it possible to stably cause hepatitis virus infection and hepatitis viral proliferation *in vitro* by infecting normal cultured human hepatocytes with a hepatitis virus. On this account, the present invention is very useful in understanding the mechanism of hepatitis virus infection and developing a good antiviral drug.

## Claims

1. A method for proliferating a hepatitis virus, the method comprising the steps of:
infecting, with the hepatitis virus, cells packed into a lumen of a hollow fiber made of a permeabilized membrane; and
culturing the cells.

2. A method for proliferating a hepatitis virus, the method comprising the steps of:
infecting cells with the hepatitis virus;
packing the cells into a lumen of a hollow fiber made of a permeabilized membrane; and
culturing the cells.

3. The method as set forth in claim 1 or 2, further comprising applying pressure or centrifugal force to the lumen of the hollow fiber into which the cells have been packed.

4. The method as set forth in any one of claims 1 through 3, wherein the cells are hepatocytes or liver-derived cells.

5. The method as set forth in claim 4, wherein the cells are HuS-E/2 cells (PERM ABP-10908).

6. The method as set forth in any one of claims 1 through 5, wherein the hepatitis virus is hepatitis B virus or hepatitis C virus.

7. A method for manufacturing a hollow fiber for culturing hepatitis virus-infected cells, the method comprising any of the following steps of:
(i) infecting, with a hepatitis virus, cells packed into a lumen of a hollow fiber made of a permeabilized membrane; and
(ii) infecting cells with a hepatitis virus and then packing the cells into a lumen of a hollow fiber made of a permeabilized membrane.

8. A hollow fiber for culturing hepatitis virus-infected cells, the hollow fiber being manufactured by a method as set forth in claim 7.

9. A method for producing a hepatitis vaccine, the method using a culture of hepatitis virus-infected cells, a hepatitis virus particle, or a constituent protein of a hepatitis virus particle, each being obtained by a method as set forth in any one of claims 1 through 6.

10. A kit for proliferating a hepatitis virus, the kit comprising a hollow fiber, made of a permeabilized membrane, whose lumen has been filled with hepatitis virus-infectable cells.

11. A method for screening a drug that inhibits hepatitis virus proliferation or hepatitis virus protein expression, the method comprising the step of culturing, together with a drug candidate substance that is to be evaluated, hepatitis virus-infected cells packed into a lumen of a hollow fiber made of a permeabilized membrane.

12. A method for screening a drug that inhibits hepatitis virus infection, the method comprising the step of culturing, together with a hepatitis virus and a drug candidate substance that is to be evaluated, hepatitis virus-infectable cells packed into a lumen of a hollow fiber made of a permeabilized membrane.

13. A kit for screening a drug that inhibits hepatitis virus infection, hepatitis virus proliferation, or hepatitis virus protein expression, the kit comprising a hollow fiber, made of a permeabilized membrane, whose lumen has been filled with hepatitis virus-infectable cells.

14. A method for screening a drug for treating a hepatitis virus-infected person, the method comprising the step of culturing, together with a drug candidate substance that is to be evaluated, cells (i) packed into a lumen of a hollow fiber made of a permeabilized membrane and (ii) infected with a hepatitis virus derived from a serum of the hepatitis virus-infected person.

15. A method for screening a drug for treating a hepatitis virus-infected person, the method comprising the step of culturing, together with (i) a hepatitis virus derived from a serum of the hepatitis virus-infected person and (ii) a drug candidate substance that is to be evaluated, hepatitis virus-infectable cells packed into a lumpen of a hollow fiber made of a permeabilized membrane.

16. A method for finding a combination of drugs most suitable for treating a hepatitis virus-infected person, the method comprising the step of culturing, together with a drug candidate substance that is to be evaluated, hepatitis virus-infected cells packed into a lumen of a hollow fiber made of a permeabilized membrane.

17. A kit for finding a combination of drugs most suitable for treating a hepatitis virus-infected person, the kit comprising a hollow fiber, made of a permeabilized membrane, whose lumen has been filled with hepatitis virus-infectable cells.

18. The method as set forth in claim 16, wherein the hepatitis virus-infected cells are hepatitis virus-infected cells derived from a serum of the hepatitis virus-infected person.

19. A diagnostic or examination method for treating a hepatitis virus-infected person, the method comprising the step of culturing, together with a drug that is to be evaluated, hepatitis virus-infected cells packed into a lumen of a hollow fiber made of a permeabilized membrane.
